# EUROPEAN PATENT APPLICATION

(11) **EP 4 005 373 A1**
(43) Date of publication of application: **01.06.2022**
(21) Application number: 20847391.8
(22) Date of filing: 24.07.2020
(51) Int. Cl.: A01K 1/015, A01K 29/00, A01K 45/00

(54) **SYSTEM AND METHOD FOR ELECTROSTERILIZING ANIMAL STALL BEDDING, THROUGH THE SYNERGISM OF PULSED ELECTRIC FIELDS AND CONDENSED ELECTROMAGNETIC WAVES APPLIED TO CORRESPONDING AGRICULTURAL EQUIPMENT/IMPLEMENTS**

(30) Priority: 26.07.2019 BR 102019015392
(71) Applicant: Duvoisin, Charles Adriano, 88330-155 Balneário Camboriu, SC (BR)
(72) Inventor: BRASIL, Edvan Alexandre De Oliveira, 88330-326 Balneário Camboriu, SC (BR); HUFF, Israel Fernandes, 88330-410 Balneário Camboriu, SC (BR); SANTIN, Marcos Ricardo, 88330-155 Balneário Camboriu, SC (BR); DUVOISIN, Charles Adriano, 88330-155 Balneário Camboriu, SC (BR)
(74) Representative: SONN Patentanwälte OG
(86) International application number: PCT/BR2020/050289
(87) International publication number: WO 2021/016693

(57) **Abstract**

The present application for invention dealt with an electrosterilization system and method for animal confinement beds, through the synergism of pulsed electric fields and condensed electromagnetic waves applied to corresponding agricultural equipment/implements, belonging to the field of methods to sterilize and sanitize animal confinement beds, through the interaction of sterilizing systems electric fields and pulsed magnetic fields and condensed electromagnetic waves. The equipment (1) being applied in agricultural and similar implements to electrosterilize animal confinement beds, through the harmonic synergism of electric fields, magnetic fields and electromagnetic waves condensed inside the tunnel (4) formed by upper electrodes (5, 5a and 5b) and lower electrodes (6, 6a and 6b), upper UVC lamps (7 and 7a) and lower UVC lamps (8 and 8a) and right polarizing electrode (10) and left polarizing electrode (11) of electromagnetic waves.

## Description

### TECHNICAL FIELD

The present application for invention relates to an electrosterilization system and method for animal confinement beds, through the synergism of pulsed electric fields and condensed electromagnetic waves applied to corresponding agricultural equipment/implements, belonging to the field of methods to sterilize and sanitize animal beds of animal confinements, through the interaction of sterilizing systems electric fields and pulsed magnetic fields and condensed electromagnetic waves.

### INTRODUCTION

The present invention application deals with a system and a method for the dynamic sterilization of animal confinement beds, developed to provide an invention capable of sanitizing the microorganisms and macroorganisms excreted and produced by animal waste in the confinement bed.

The term used here as "bed" is all material distributed in a shed or barn to serve as a bed for the animals, more specifically the material that, remaining on the floor of a facility, will receive excretion, feed remains, feathers, hair and the like.

### PRIOR-ART

Cleaning processes for animal confinement beds, to date, are still based on burning by gas flames, application of lime (calcium oxide) and toxic chemical agents based on insecticides and fungicides, in order to contain microorganisms and disease-promoting macroorganisms.

One of the great challenges to be overcome is the containment of beetles *Alphitobius diaperinus* that lodge in the confinement beds, a serious problem and still without a definitive solution to eliminate these insects.

Another challenge is to contain the bacteria of *Salmonella SSP,* which is becoming more resistant to the effects of trivial chemicals.

Therefore, in the prior-art there is a wide variety of solutions that provide for the sanitization of confinement beds, but all with important limitations, since the invention proposed in this patent allows a totally viable and effective way to control the transmitters of the diseases in question, such as the one presented in document BR102016021478 5A2 of 2016, which describes a self-cleaning system with UV-C sterilization radiation for agriculture, in order to keep the animal's environment free of bacteria, viruses, fungi and other harmful agents, through a blade of effluent generated in the stall of the pigsty/stall that will be irradiated by UV-C germicidal lamps arranged in highly reflective aluminum gutters interconnected by feed reactors and wires protected by silicone hoses.

The document above reports the concern of inventor Carla Brenner to sterilize the confinement bed with a system based on UV-C (Ultra-violet-C) waves, which is done using a static method, a detail that is quite different from the system claimed here which is designed to use pulsed electric fields with the use of electrodes that enable the technology intended here, which is the condensation of electromagnetic waves.

The author's own document BR102018068980-0 of 2018 describes a "system and method of condensation of electromagnetic waves through modulated electric and magnetic fields and corresponding equipment" that reports the possibility of condensation of electromagnetic waves through properly aligned electric and magnetic fields, technology whose subject won the 2018 Nobel Prize in Physics.

Document BR1120160120396A2 of 2013 describes a process and system for cleaning and sanitizing agro-industrial farms such as production farms, breeding farms and incubators for poultry, swine and eggs, using a system comprising an autonomous mobile unit that includes a production unit of foam for the application of a specialized formulation of detergent for livestock use and a pressure hydrowashing unit for pre-rinsing and rinsing, as well as the cleaning and sanitization process using the system comprising the autonomous unit, enabling an increase in the cleaning efficiency and productivity, and reducing the mortality rate of animals.

The above document shows the concern of sanitizing the confinement bed using a chemical method based on detergents/surfactants and also toxic chemical agents, with the attempt to eliminate living organic agents that produce diseases in these beds in question, however the author already reports to the attempt, knowing the current deficiencies in the sanitization processes of contemporary confinement beds, as well as how this invention is totally different from the patent proposed here, which deals with sterilizing physical actions, through condensed electromagnetic waves and pulsed electric and magnetic fields.

Patent document BR102013018666-0A2 of 2013 describes an equipment and continuous process for treating waste, food and other materials, by cold plasma sterilization, deep drying, compaction and packaging, which comprises deep drying equipment, plasma sterilization cold and solid waste packaging intended to fulfill the functions of liquid withdrawal, plasma sterilization and waste packaging.

This invention mentioned above uses cold plasma to sterilize waste, but it is a static equipment, different from the invention claimed here, which uses a condenser system of electromagnetic waves applied to equipment capable of sterilizing litter for poultry and other animals, including being a equipment capable of being coupled to tractors or versatile automobiles, to work within the confinement areas even with the presence of animals in the place to be sterilized.

Patent document BR102019014977-9, by the same author, describes an appliance and/or aggregating module for electropasteurizing and electrosterilizing food and utensils, through harmonic synchrony of electric fields, magnetic fields and electromagnetic wave condenser system, with the ability to preserve their organoleptic properties, thus allowing food to be sanitized before being ingested.

Although this patent mentioned above is a technology directed to an appliance, it differs from the proposal of the patent claimed here, because it does not present a lateral system for polarizing electromagnetic waves, because with this detail there will be a distinct effect of the condensation of these electromagnetic waves, thus providing an acceleration of the sterilizing process in question.

Thus, patent documents BR1020160214785A2, BR102018068980-0, BR1120160120396A2, BR102019014977-9 and BR102013018666-0 basically use only electromagnetic radiation that works superficially, and the chemical means used intoxicate the confined animals, in addition to not being complete sterilizers.

Thus, the invention claimed here differs from the others mentioned above precisely because it uses a specific sterilizing innovation to electrosterilize animal confinement beds, through the synergism of pulsed electric fields and condensed electromagnetic waves applied to an equipment that can be coupled in a variety of models of agricultural implements.

The equipment intended here performs the forced passage of the bed inside the equipment so that it receives the condensation of specific electromagnetic waves, generated by pulsatile electric and magnetic fields, with the condensation of electromagnetic waves.

The system claimed here, when applied to equipment, can be placed in any type of installation used in livestock and has as main objective the development of a self-disinfecting and sterilizing system adaptable in old or new installations.

That is, the invention proposed here is aimed at the condensation of electromagnetic waves, through a polarizing method by lateral polarization systems and thus providing an accelerating effect of the condensation of electromagnetic waves, providing even more penetrating and sterilizing ultraviolet waves, such as for example UV-C, which will become more energetic, creating UV-Z waves and also with even more lethal effects to micro and macro-organisms, allowing a system and method capable of dynamically sterilizing the confinement beds in question.

This system and method is designed to be installed in a corresponding equipment, which provides the passage of this confinement bed between the condensed electromagnetic waves created by the specific electrodes and electromagnetic wave generator lamp parallelly aligned at the top and bottom, or in another form of right side and left side mounting.

### DESCRIPTION OF THE FIGURES

The attached drawings show the electrosterilization system and method of animal confinement beds, through the synergism of pulsed electric fields and condensed electromagnetic waves applied to the corresponding agricultural equipment/implements, which together with the numerical references detailed below, make it understood more easily, although this invention can vary in many different constructive forms, always customized for each application, not shown in the drawings that will be described in detail herein and forms for carrying out said invention.

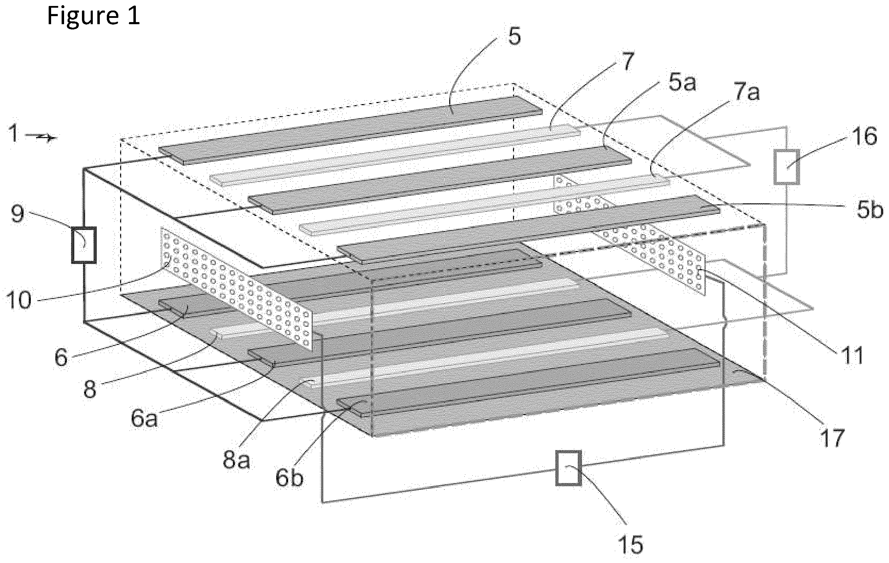

Figure 1 presents a partial overview showing the assembly sequence and positioning for increasing the number of upper electrodes, lower electrodes, upper UVC lamp and lower UVC lamp connected to an exclusive voltage source.

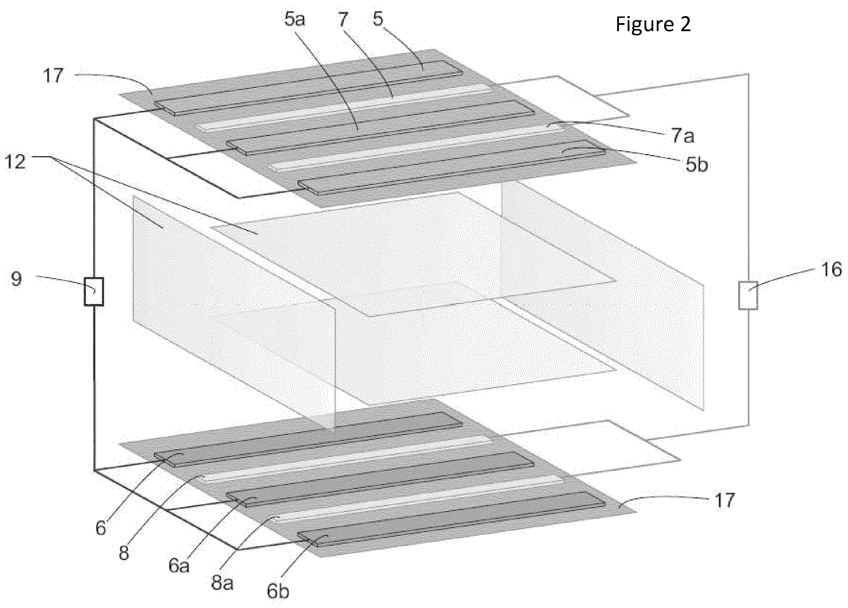

Figure 2 presents a partial overview showing the parallel positioning of the upper electrodes in relation to the lower electrodes, of the upper UVC lamps aligned parallel with the lower UVC lamps, mounted on the electrical insulators and internally protected by the platforms made of translucent materials.

Figure 3 shows a view of the positioning of the upper electrodes aligned parallel to the lower electrodes and the upper UVC lamps aligned parallel to the lower UVC lamps, thus forming the upper cassette and lower cassette identical in their assembly and parallel alignment.

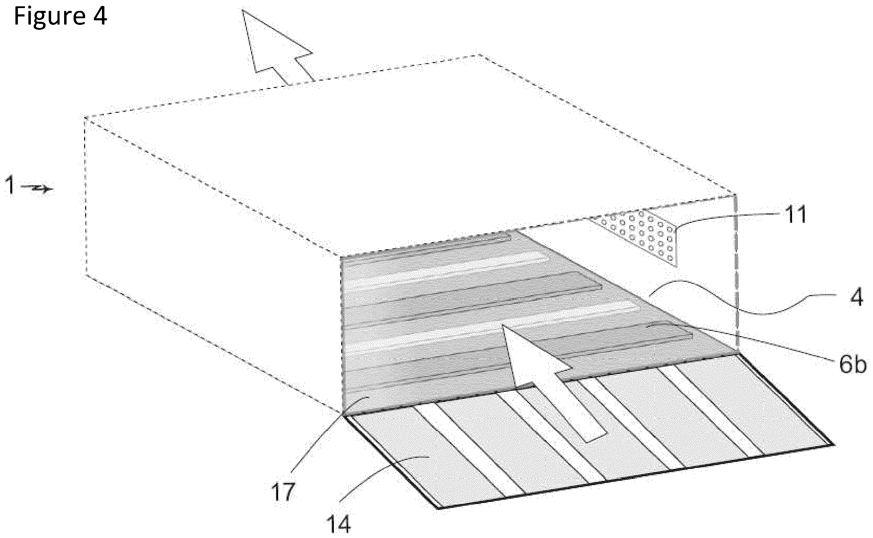

Figure 4 shows, as an example, one of the possible variations of equipment assembly to be mounted on an agricultural implement or any other vehicle for its displacement within an animal confinement area, for the forced entrance of the bed indicated by arrow into the longitudinal tunnel, for its exit from inside the tunnel, already sterilized by the condensed waves.

### DETAILED DESCRIPTION OF THE DISCLOSURE

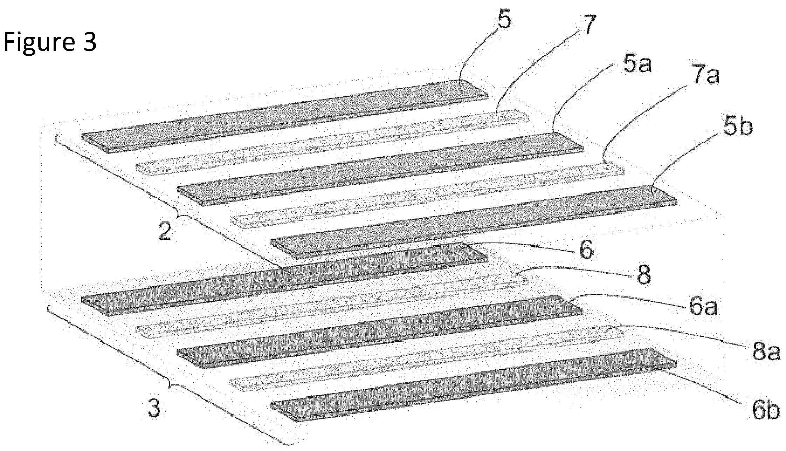

A confinement bed sterilizer system by the interaction of electric and magnetic fields and condensed electromagnetic waves, through an upper cassette (2) and a lower cassette (3) identical in their assembly and parallel alignment with each other (figure 3); the upper cassette (2) being composed of at least one pair of upper electrodes (5 and 5a), which must always be located in parallel with the lower electrodes (6 and 6a), thus avoiding displacement of the "curtain" of the cold plasma, normally created due to the ionization of air or local gases existing in this medium from developed electric and magnetic fields.

The amount of upper cassette (2) and lower cassette (3) assembled in each equipment may vary according to the need in its production, but always following a construction order that starts with the upper cassette (2) formed by the upper electrode (5) laterally aligned with the upper UVC lamp (7) and in the lateral sequence by another upper electrode (5a) and so on, being interspersed by another upper UVC lamp (7a) laterally aligned by another upper electrode (5b), always respecting and identical to its parallel assembly and aligned with the lower cassette (3).

The lower cassette (3) is formed by a lower electrode (6) laterally aligned by a lower UVC lamp (8) and in the sequence laterally aligned by another lower electrode (6a) and so on, being interspersed by another lower UVC lamp (8a) laterally aligned by another lower electrode (6b) (figure 3) and so on, so that they intervene in the energetic state of the electromagnetic waves emitted by the UVC lamps.

The operation of the upper cassette (2) and lower cassette (3) in parallel alignment with each other generates the condensation of the desired condensed electromagnetic waves.

The alignment of the upper cassette (2) with the lower cassette (3) occurs following the perfect parallel alignment between the upper electrode (5) aligning parallel with the lower electrode (6), upper UVC lamp (7) aligned parallel with the lamp lower UVC lamp (8), upper electrode (5a) aligning parallel with lower electrode (6a), upper UVC lamp (7a) aligned parallel with lower UVC lamp (8a), upper electrode (5b) aligning parallel with lower electrode (6b) and so on, being able to hold several electrodes and lamps, always following the same assembly sequence between the upper cassette (2) and the lower cassette (3).

The upper UVC lamps (7 and 7a) aligned parallel with the lower UVC lamps (8 and 8a) are electrically powered by a voltage source (16).

The equipment (1) can be coupled to a drivable vehicle, such as agricultural implements with traction or with its own traction, capable of carrying out the process of passing the bed in question through a longitudinal sterilizing tunnel (4), which has one or more upper cassettes (2) and lower cassette (3) forming electric and magnetic fields and condensed electromagnetic waves.

The upper cassette (2) formed by upper electrodes (5, 5a and 5b) and so on, always obeying the parallel and analogous assembly pattern with the lower cassette (3) formed by lower electrodes (6, 6a and 6b) and so on, connected to different high voltage sources (9) from 1 V to 100 GV, preferably 50 kV and with a frequency of 50 Hz to 800 GHz, preferably 10 MHz.

The high voltage source (9) for the upper electrodes (5, 5a, 5b) successively must always be positioned and interconnected in a parallel and analogous way with the lower electrodes (6, 6a, 6b) successively for the upper cassette assembly (2) and lower cassette (3).

The high voltage sources (9) can produce direct or alternating electric currents, they can also be pulsed, with ideal voltages and frequencies to generate cold plasma, as well as an ideal medium to allow the condensation of electromagnetic waves.

In this environment of "curtains" of electrons and electric and magnetic fields created, a beam of electromagnetic waves is emitted, created by the upper UVC lamp (7) and lower UVC lamp (8) preferably where these electromagnetic waves will condense due to polarization created by the right polarizer (10) and left polarizer (11) of attraction and repulsion thereof.

Electromagnetic waves are made of interspersing electric and magnetic pulses and thus if we synchronize the pulses so that there is a harmony of the electric and magnetic fields generated by the upper electrodes (5, 5a and 5b) and lower electrodes (6, 6a and 6b), in question, which will intervene in the energetic state of the electromagnetic waves emitted by the upper UVC lamps (7 and 7a) and lower UVC lamps (8 and 8a) present in each upper cassette (2) and lower cassette (3).

The high voltage sources (15) exclusive to the right polarizer (10) and left polarizer (11) may operate on industrial, domestic or automotive or other battery power.

The high voltage sources (15) can produce direct or alternating electric currents, they can also be pulsed, with ideal voltages and frequencies to generate cold plasma, as well as an ideal medium to allow the condensation of the electromagnetic waves created by the superior UVC lamps (7) and lower UVC lamp (8), preferably UV-C, providing more energetic waves, such as: UV-D e UV-F, etc.

In order to enable the passage of condensed ultraviolet light, platforms (12) made of materials such as glass based on materials translucent to condensed electromagnetic waves are used, preferably UV-C condensed to UV-D or UV-F.

Between the alignment of the upper cassette (2) and lower cassette (3) there must be a longitudinal tunnel (4) for the passage of the confinement bed, which will provide the process of sanitization and electrosterilization of the confinement bed.

In order for it to pass through the tunnel (4) to be processed in the tunnel, mechanical tractors (14) will be used, such as helical screws, rotary brooms, fixed shovels, mats, among others, whose function will be to promote the continuous and automatic forced flow of the passage of the bed inside the tunnel (4).

Electrical insulating systems (17) provide electrical isolation between the upper electrodes (5, 5a and 5b) and lower electrodes (6, 6a and 6b), upper UVC lamps (7 and 7a) and lower UVC lamps (8 and 8a) present in each upper cassette (2) and lower cassette (3), preventing the escape of electrical currents to the metallic parts of the equipment (1).

Security systems such as a set of audible or visual alarms to monitor the correct functioning of the equipment (1) may be added as needed in its manufacture and sale.

The equipment (1) as described above will be composed of several upper electrodes (5, 5a and 5b) and lower electrodes (6, 6a and 6b), this quantity can be increased at any time according to the need in its project, so that these pairs must be interspersed in a parallel and analogous way and always connected to the same specific high voltage source (9).

In the upper cassette (2) and lower cassette (3) sterilizers there must be sources emitting electromagnetic waves produced by the upper UVC lamp (7) and lower UVC lamp (8), which will be condensed by the harmonic and interposed effects of electric fields and magnets in question.

There must also be a system of polarizing electrodes on each side, formed by a right polarizer (10) and a left polarizer (11) of electromagnetic waves, this polarization will occur due to the state of the electric current generated by its continuous high voltage source (15) which also can be pulsed, generating a constant electric and magnetic field and thus facilitating the condensation of the electromagnetic waves emitted in this system of electrosterilization.

This condensation generated from electromagnetic waves emitted in this system of electrosterilization performs total sterilization, including the most internal parts of this confinement bed, more efficiently, overcoming the problems presented in the prior-art, applied to sanitize the confinement beds.

The method for sanitizing and sterilizing the confinement bed according to this invention is capable of being carried out by a system comprising at least the steps of sterilizing the confinement bed; mechanizing of the passage of this confinement bed through the longitudinal tunnel (4) which has a right polarizer (10), a left polarizer (11), an upper cassette (2) and lower cassette (3) sterilizer by electric and magnetic fields and electromagnetic waves condensed.

Inside the longitudinal tunnel (4) is where the synergism of pulsed electric fields and condensed electromagnetic waves occurs for the electrosterilization of the confinement bed, during its continuous passage.

## Claims

1. System and method of electrosterilization of animal confinement beds, by means of the synergism of pulsed electric fields and condensed electromagnetic waves applied to corresponding agricultural equipment/implements, **characterized by** a system of electrosterilization of animal confinement beds, through the harmonic synergism of electric fields, magnetic fields and condensed electromagnetic waves, applied to equipment (1) applied in agricultural implements and similar to electrosterilize animal confinement beds, through harmonic synergism of electric fields, magnetic fields and electromagnetic waves condensed inside the longitudinal tunnel (4) formed by at least a pair of upper electrodes (5 and 5a) interspersed by an upper UVC lamp (7) forming an upper cassette (2), aligned parallel with the lower cassette (3) formed by at least a pair of lower electrodes (6 and 6a) interspersed with a lower UVC lamp (8), together with polarizing electrodes formed by a right polarizer (10) and a left polarizer (11) of electromagnetic waves.

2. System and method of electrosterilization of animal confinement beds, through the synergism of pulsed electric fields and condensed electromagnetic waves applied to corresponding agricultural equipment/implements, according to claim 1, **characterized by** the amount of upper cassette (2) and lower cassette (3) assembled in each equipment (1) can vary, always following a construction order that starts with the upper electrode (5) laterally aligned and separated by an upper UVC lamp (7) which is laterally aligned with another upper electrode (5a) and so on, being interspersed by another upper UVC lamp (7a) laterally aligned by another upper electrode (5b), always following and identical to the parallel assembly on the opposite side on the opposite side in relation to the alignment with the lower electrode (6) followed laterally by a lower UVC lamp (8) laterally aligned by another lower electrode (6a) and so on interspersed by another lower UVC lamp (8a), laterally aligned by another lower electrode (6b) successively in this order of assembly.

3. System and method of electrosterilization of animal confinement beds, through the synergism of pulsed electric fields and condensed electromagnetic waves applied to corresponding agricultural equipment/implements, according to claim 1, **characterized by** the parallel alignment between the upper cassette (2) with the lower cassette (3) following the perfect alignment between the upper electrode (5) aligned parallel with the lower electrode (6), upper UVC lamp (7) aligned parallel with the lower UVC lamp (8), upper electrode (5a) aligned parallel with lower electrode (6a), upper UVC lamp (7a) aligned parallel with lower UVC lamp (8a), upper electrode (5b) aligned parallel with lower electrode (6b) and so on, being able to hold several electrodes and lamps, always following the same assembly sequence between the upper cassette (2) and the lower cassette (3).

4. System and method of electrosterilization of animal confinement beds, through the synergism of pulsed electric fields and condensed electromagnetic waves applied to corresponding agricultural equipment/implements, according to claim 1, **characterized by** the polarizing electrodes being formed by a right polarizer (10) and a left polarizer (11) of electromagnetic waves, fed by electric current generated by a continuous and pulsed high voltage source (15).

5. System and method of electrosterilization of animal confinement beds, through the synergism of pulsed electric fields and condensed electromagnetic waves applied to corresponding agricultural equipment/implements, according to claim 1, **characterized by** the upper UVC lamps (7 and 7a) aligned parallel with the lower UVC lamps (8 and 8a) being electrically powered by a single voltage source (16).

6. System and method of electrosterilization of animal confinement beds, through the synergism of pulsed electric fields and condensed electromagnetic waves applied to corresponding agricultural equipment/implements, according to claim 1, **characterized by** the upper electrodes (5, 5a, 5b) successively being positioned and interconnected in a parallel and analogous manner with the lower electrodes (6, 6a, 6b) successively for the upper cassette assembly (2) and lower cassette (3) electric fields and magnetic fields generators electrically powered by specific high voltage sources (9) operating between 1 V to 100 GV, preferably 50 kV and with a frequency of 50 Hz to 800 GHz, preferably 10 MHz.

7. System and method of electrosterilization of animal confinement beds, through the synergism of pulsed electric fields and condensed electromagnetic waves applied to corresponding agricultural equipment/implements, according to claim 1, **characterized by** comprising the equipment (1) presenting a mechanical traction mechanism (14) that leads to continuous, forced and automatic passage of the bed inside the tunnel (4) for electrosterilization by the condensates in UV-Z penetrating waves.

8. System and method of electrosterilization of animal confinement beds, through the synergism of pulsed electric fields and condensed electromagnetic waves applied to corresponding agricultural equipment/implements, according to claim 1, **characterized by** the number of upper electrodes (5, 5a and 5b) and so on, lower electrodes (6, 6a and 6b) and so on using only one high voltage source (9); upper UVC lamps (7 and 7a) and lower UVC lamps (8 and 8a) and so on using only one voltage source (16); and right polarizer (10) and left polarizer (11) using only one high voltage source (15).

9. System and method of electrosterilization of animal confinement beds, through the synergism of pulsed electric fields and condensed electromagnetic waves applied to corresponding agricultural equipment/implements, according to claim 1, **characterized by** the high voltage source (9) and voltage source (16) being capable of producing pulsed direct currents or alternating currents with high electrical voltages from 1 kV to 100 GV, with frequencies varying from 50 Hz to a maximum possible limit.

10. System and method electrosterilization of animal confinement beds, through the synergism of pulsed electric fields and condensed electromagnetic waves applied to corresponding agricultural equipment/implements, according to claim 1, **characterized in that** equipment 1 has electrical insulating systems (17), providing electrical isolation between the upper electrodes (5, 5a and 5b) and lower electrodes (6, 6a and 6b), upper UVC lamps (7 and 7a) and lower UVC lamps (8 and 8a) present in each upper cassette (2) and lower cassette (3).

11. System and method electrosterilization of animal confinement beds, through the synergism of pulsed electric fields and condensed electromagnetic waves applied to corresponding agricultural equipment/implements, according to claim 1, **characterized by** the equipment 1 internally present platforms (12) made of translucent materials for the passage of condensed electromagnetic waves UV-C, condensed for UV-D or UV-F.

12. System and method electrosterilization of animal confinement beds, through the synergism of pulsed electric fields and condensed electromagnetic waves applied to corresponding agricultural equipment/implements, according to claim 1, **characterized by** the system of electrosterilization of animal confinement bed, through the harmonic synergism of electric fields, magnetic fields and condensed electromagnetic waves, applied to equipment (1), occurs inside the longitudinal tunnel (4) that has a right polarizer (10), a left polarizer (11), an upper cassette (2) and a lower cassette (3) sterilizer by electric and magnetic fields and condensed electromagnetic waves.
